# EUROPEAN PATENT APPLICATION

(11) **EP 1 900 327 A1**
(43) Date of publication of application: **19.03.2008**
(21) Application number: 07017023.8
(22) Date of filing: 30.08.2007
(51) Int. Cl.: A61B 8/00

(54) **An ultrasonic probe, an ultrasonic diagnostic device, an ultrasonic-probe-monitoring system, and a method of state management of the ultrasonic probe**

(30) Priority: 11.09.2006 JP 2006245560
(71) Applicant: KABUSHIKI KAISHA TOSHIBA, Tokyo (JP); Toshiba Medical Systems Corporation, Otawara-shi, Tochigi (JP)
(72) Inventor: Miyajima, Yasuo, Otawara-shi Tochigi (JP)
(74) Representative: Kramer - Barske - Schmidtchen

(57) **Abstract**

An ultrasonic probe comprises: an internal power source configured to be capable of supplying electrical power to the inside in a state in which the ultrasonic probe is detached from an ultrasonic diagnostic device; and a state-management part configured to be fed with power from the internal power source and manage the state of an ultrasonic probe's main body. Consequently, it becomes possible to manage in real time the state of the ultrasonic probe in non-use. The state management in non-use allows for the factors behind abnormality occurrences to be specified and for secure storage management to be accomplished.

## Description

### BACKGROUND OF THE INVENTION

### 1. Field of the Invention

The present invention relates to a technique of management of an ultrasonic probe.

### 2. Description of the Related Art

In ultrasonic diagnostics, ultrasonic waves are transmitted to the inside of a subject body, and diagnostic information such as a tomographic image of the inside of the subject body is generated based on waves reflected from the inside of the subject body. Transmission/reception of the ultrasonic waves to/from the subject is performed via an ultrasonic probe.

The ultrasonic probe has an ultrasonic transducer having a piezoelectric effect of reversibly converting signal voltage and sonic waves. The ultrasonic transducer produces ultrasonic waves when voltage is applied thereto, and generates electrical signals when receiving reflected waves. The ultrasonic probe is connected to an ultrasonic diagnostic device when used for ultrasonic diagnostics. The ultrasonic probe is connected to and powered by the ultrasonic diagnostic device. In addition, the ultrasonic probe is connected to the ultrasonic diagnostic device, thereby transmitting/receiving, control signals for applying voltage to the ultrasonic transducer and electrical signals generated by the ultrasonic transducer.

In general, the ultrasonic probe has a cable for transmitting signals. However, since the ultrasonic probe is grasped and handled by an operator, the cable may be an impediment to diagnostics, and cable breakage may cause electrical leakage, coolant leakage and the like. Therefore, a technique of an ultrasonic probe that comprises an internal power supply and a radio communication part and is powered by the internal power supply to transmit/receive signals using the radio communication part is disclosed (*cf*. e.g. Publication of Japanese Unexamined Patent Application JP-A 2003-10177). This technique makes it possible to transmit/receive signals for ultrasonic diagnostics wirelessly, thereby eliminating the need for the cable.

Meanwhile, this ultrasonic probe has a complex inner structure and is vulnerable to shock, and insulation measures are taken to prevent electrical leakage to a subject. Therefore, an operator needs to pay full attention when handling the ultrasonic probe. Moreover, the ultrasonic probe may be contaminated with a virus after used for a patient infected by the virus. Hence, the operator needs to take care when handling the ultrasonic probe. Use of the defective ultrasonic probe may result in generation of incorrect diagnostic information and insufficient diagnosis. Use of the ultrasonic probe contaminated with the virus for diagnosis may cause infection of other patients and the operator. Furthermore, since ultrasonic probes are expensive, sufficient precautions must be made against thefts and the like.

Accordingly, it is necessary to adequately manage the state of the ultrasonic probe, such as what physical irritations the ultrasonic probe has received, how the ultrasonic probe is currently being handled, and how the ultrasonic probe is stored. It is important to manage the state of the ultrasonic probe in non-use, rather than in use. This is because, oftentimes, the ultrasonic probe is dropped or the cable is stepped on at times such as when the probe is being conveyed to a safekeeping room after ultrasonic diagnosis ends, when the probe is being kept in the safekeeping room, when the probe is being carried out from the safekeeping room for a sterilization process, when the probe is being carried out from the safekeeping room for maintenance, and when the probe is being carried out from the safekeeping room for use in diagnosis.

Conventionally, a serviceman subjects the ultrasonic probe to an advanced inspection test during routine inspections, but daily inspections are nothing more than simple inspection tests. In the daily inspections, it is difficult to find abnormalities that are not manifested in the ultrasonic probe's appearance, because the only tests that can be performed are visual tests, simple image tests, and the like. Since factors behind abnormalities occurring while the ultrasonic probe is stored or conveyed are not specified in either the routine inspections or the daily inspections, it is impossible to analyze possible abnormalities. Further, leaving the factors of the abnormality occurrences unspecified makes it difficult to find failures. If the factors of the abnormality occurrences cannot be specified, it is difficult to determine what kind of handling causes abnormalities, and it is hard to handle appropriately.

In other words, in order to rationalize the pursuance of abnormality occurrences and handling, it is necessary to manage in real time the state of the ultrasonic probe particularly in non-use (i.e. in the state of being unconnected to the ultrasonic diagnostic device).

### SUMMARY OF THE INVENTION

The present invention has been achieved in consideration of the above circumstances, and an object of the present invention is to provide a technique of managing the state of an ultrasonic probe in non-use unconnected to an ultrasonic diagnostic device.

In a first aspect of the present invention, an ultrasonic probe comprises a state-management part configured to manage a state of the ultrasonic probe, and an internal power supply configured to be capable of supplying electrical power to the state-management part in a state in which a connector is detached from an ultrasonic diagnostic device. Consequently, it becomes possible to manage in real time the state of the ultrasonic probe in non-use. The state management in non-use allows for the factors of abnormality occurrences to be specified, and also allows for secure storage management to be accomplished.

In a second aspect of the present invention, the state-management part includes a sensor configured to sense physical irritations received by the ultrasonic probe, and a detector configured to, based on a result of sensing by the sensor, detect the physical irritations of a predetermined level or more. Consequently, it is possible to detect the physical irritations of the predetermined level or more received by the ultrasonic probe in non-use, and it becomes possible to specify the factors of abnormality occurrences.

In a third aspect of the present invention, the state-management part notifies reception of physical irritations of a predetermined level or more, by sound, light or display. Consequently, a handler can comprehend what kind of handling may cause abnormalities, and therefore, can learn appropriate handling.

In a fourth aspect of the present invention, the state-management part further includes a communication part configured to, when the physical irritation of the predetermined level or more is detected, output a signal to cancel signal transmission/reception to the ultrasonic diagnostic device when the connector is connected to the ultrasonic diagnostic device. Consequently, it is possible to prevent the ultrasonic probe from being used in diagnosis as an operator is unaware of the abnormality occurrence, and it is possible to prevent false diagnoses with inaccurate diagnostic information or re-diagnoses.

In a fifth aspect of the present invention, the state-management part includes a communication part configured to receive handling information of the ultrasonic probe from an external device. Consequently, it becomes possible to immediately comprehend handling information, such as whether the ultrasonic probe is currently waiting for sterilization or maintenance.

In a sixth aspect of the present invention, the state-management part includes a sensor configured to measure proximity of an object, and a notifying part configured to, when the communication part receives the predetermined handling information and the sensor measures the proximity of the object, give a warning by sound, light or display. Consequently, it becomes possible to prevent disasters caused by the use of an ultrasonic probe that may adversely affect a human body.

In a seventh aspect of the present invention, the state-management part includes a radio-communication part configured to communicate by radio with an external device, and a notifying part configured to, when radio communication between the radio-communication part and the external device becomes disconnected, give a warning by sound, light or display. Consequently, it becomes possible, when the ultrasonic probe is being taken out without permission, to give a warning to a person taking out the probe, and an antitheft effect is produced.

In an eighth aspect of the present invention, the state-management part includes a radio-communication part configured to receive a calling signal from an external device, and a notifying part configured to, when the radio-communication part receives the calling signal, notify a position of the ultrasonic probe, by sound, light or display. Consequently, it is possible to easily select a desired ultrasonic probe from among a plurality of ultrasonic probes, and it is possible to diagnose by using an ultrasonic probe 1 having no abnormalities

In a ninth aspect of the present invention, the ultrasonic probe further comprises an activation controller configured to control timing of power feeding to the state-management part from the internal power supply. Consequently, it is possible to hold down power consumption of the internal power supply and achieve high convenience.

### BRIEF DESCRIPTION OF THE DRAWINGS

Fig. 1 shows the appearance of an ultrasonic probe.
Fig. 2 schematically shows a configuration relating to transmission/reception of ultrasonic waves by the ultrasonic probe.
Fig. 3 shows part of a circuit configuration of the ultrasonic probe.
Fig. 4 shows a configuration of a sensor placed in the ultrasonic probe.
Fig. 5 shows a functional configuration of the ultrasonic probe.
Fig. 6 is a flowchart showing an operation by a detector provided in the ultrasonic probe.
Fig. 7 is a flowchart showing an operation of writing handling information by an information-writing part provided in the ultrasonic probe.
Fig. 8 is a flowchart showing an operation of providing physical-irritation information and handling information, by a state-informing part provided in the ultrasonic probe.
Fig. 9 schematically shows an example of a screen of an external apparatus, on which strings corresponding to the physical-irritation information or handling information are displayed.
Fig. 10 is a flowchart showing an operation of canceling data communication by a connection-canceling part provided in the ultrasonic probe.
Fig. 11 is a pattern diagram showing an example of a screen of the ultrasonic diagnostic device having received a cancel signal.
Fig. 12 is a flowchart showing an operation of warning against takeout by a takeout-warning part provided in the ultrasonic probe.
Fig. 13 schematically shows a state in which the ultrasonic probe is warning against an unauthorized takeout.
Fig. 14 is a flowchart showing an operation of warning against proximity by a proximity-warning part provided in the ultrasonic probe.
Fig. 15 schematically shows a state in which the ultrasonic probe is warning against proximity.
Fig. 16 is a flowchart showing an operation of informing a position by a position-informing part provided in the ultrasonic probe.
Fig. 17 schematically shows a state in which the ultrasonic probe is informing a position.

### DETAILED DESCRIPTION OF THE EMBODIMENT

Hereinafter, a preferred embodiment of the ultrasonic probe according to the present invention will be specifically explained referring to the drawings.

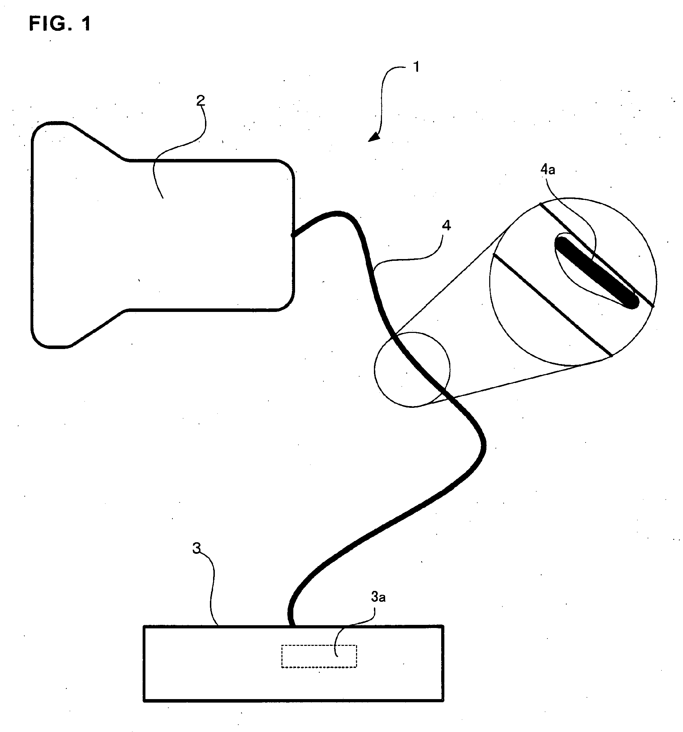

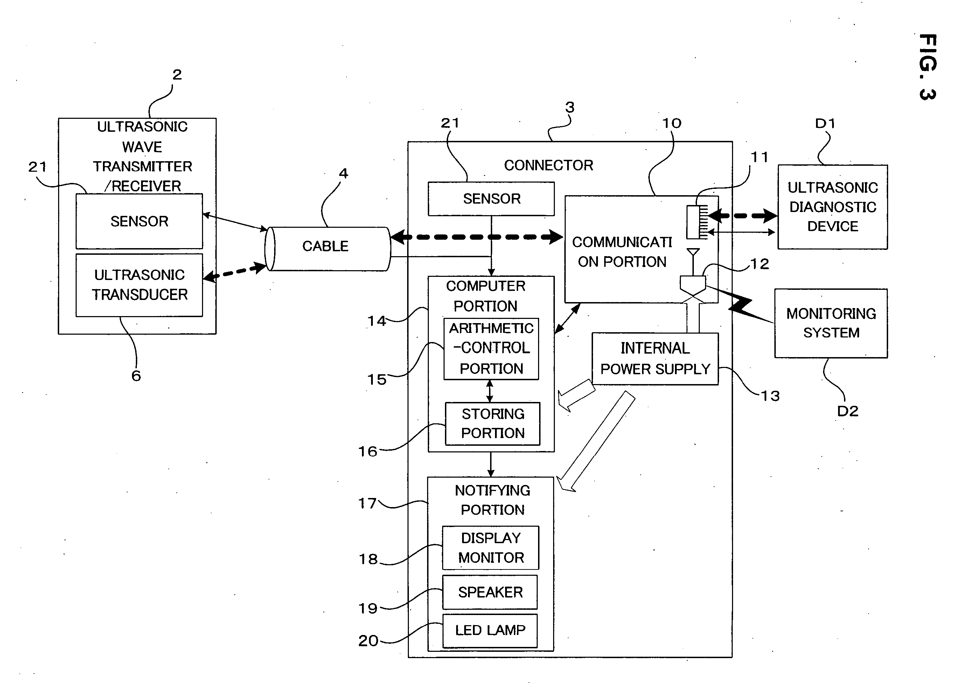

Fig. 1 is an appearance view showing the ultrasonic probe. An ultrasonic probe 1 transmits ultrasonic waves to a subject, and receives reflected waves generated by inconsistencies of acoustic impedance in the subject body. The ultrasonic probe 1 is connected to an ultrasonic diagnostic device D1 (*cf.* Fig. 3) when used. The received reflected waves are converted into electrical signals, and these electrical signals are outputted to the ultrasonic diagnostic device D1. The ultrasonic diagnostic device D1 executes image processing of the inputted electrical signals and displays an image of the inside of the subject body.

This ultrasonic probe 1 comprises an ultrasonic wave transmitter/receiver 2, a connector 3, and a cable 4. The ultrasonic wave transmitter/receiver 2 transmits/receives ultrasonic waves. The ultrasonic probe 1 is used in a state in which the ultrasonic wave transmitter/receiver 2 is held by hand. The connector 3 is connected to the ultrasonic diagnostic device D1 and mediates electrical signals that are inputted into or outputted from the ultrasonic wave transmitter/receiver 2. The cable 4 mediates conveyance of electrical signals between the connector 3 and the ultrasonic wave transmitter/receiver 2. One end of the cable 4 is mounted onto the rear face of the ultrasonic wave transmitter/receiver 2, and the other end of the cable 4 is mounted onto the connector 3. A conductive tube 4a is provided in the cable 4. The conductive tube 4a allows the flow of coolant for cooling the ultrasonic wave transmitter/receiver 2, which is heated by oscillations of ultrasonic waves. A pump 3a is provided in the connector 3. Connection of the conductive tube 4a and the pump 3a allows circulation of the coolant.

The ultrasonic probe 1 is used in a state in which the connector 3 is connected to a connecting portion of the ultrasonic diagnostic device D1. When not in use, the ultrasonic probe is detached from the ultrasonic diagnostic device D1, and stored or subjected to maintenance. The ultrasonic probe 1 comprises a part configured to manage the state of the ultrasonic probe 1. "To be in non-use" includes "to be stored," "to be conveyed," and "to be subjected to maintenance."

The part managing the state manages the state of the ultrasonic probe in non-use, such as what physical irritations the ultrasonic probe has received, how the ultrasonic probe is currently being handled, and how the ultrasonic probe is stored. The physical irritations include physical irritations from the external environment and physical irritations produced inside the probe. The physical irritation is shock, temperature, pressure, and changes in electrical resistance. Handling of the ultrasonic probe 1 refers to the way of handling the ultrasonic probe in non-use, such as when waiting for sterilization, when waiting for maintenance, and when being lent out to another section. The presence of the ultrasonic probe 1 includes the position of the ultrasonic, probe 1, and disappearance thereof due to takeout from a position where the probe should be present. The state-management part actively and passively manages the state of the ultrasonic probe 1, and informs an external device and a human of the management result or a warning according to the management result. The management result and the warning are given to the external device by data communication, and given to the human by a method stimulating acoustically or visually. The external device is a monitoring system D2 *(cf.* Fig. 3) capable of communicating with the ultrasonic probe 1, and includes, other than equipment capable of radio communication, the ultrasonic diagnostic device D1 connected to the ultrasonic probe 1 by wire.

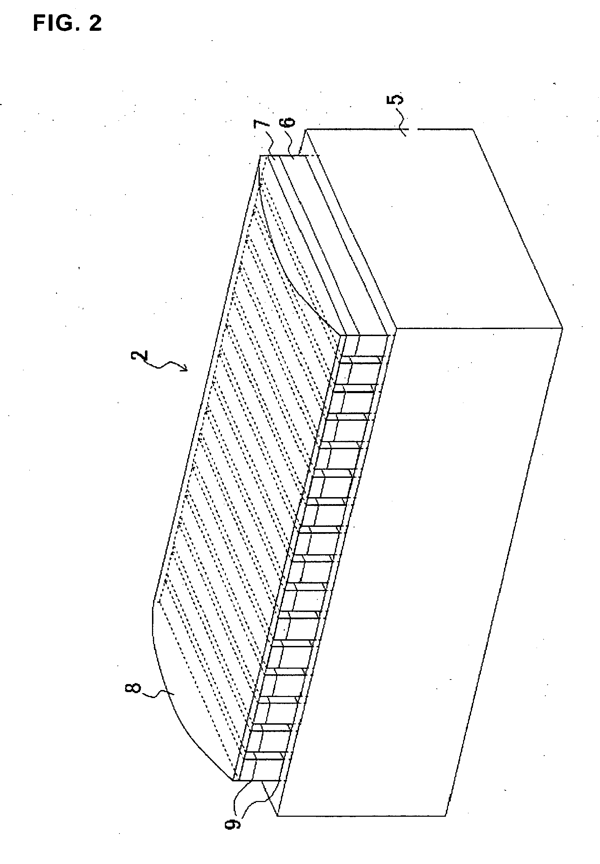

Fig. 2 shows a typical configuration relating to the transmission/reception of ultrasonic waves by the ultrasonic probe 1. The ultrasonic wave transmitter/receiver 2 is configured by accumulating an ultrasonic transducer 6, an acoustic-matching layer 7 and an acoustic lens 8 in this order on one surface of a backing material 5. The ultrasonic transducer 6 is sandwiched between an electrode pair 9. The backing material 5, the ultrasonic transducer 6, the acoustic-matching layer 7, the acoustic lens 8, and the electrode pair 9 are housed in a probe case. The tip of the probe case comes in contact with the surface of the body of the subject when the ultrasonic probe is in use. These internal configurations are housed inside the probe case so that the acoustic lens 8 is located on the side coming in contact with the surface of the body of the subject.

The ultrasonic transducer 6 is an element having a piezoelectric effect allowing reversible conversion between acoustics and electricity. Piezo-ceramic material such as lead zirconate titanate Pb (Zr, Ti) O₃, lithium niobate (LiNbO₃), barium titanate (BaTiO₃), and lead titanate (PbTiO₃) is employed. The ultrasonic transducers 6 are apposed on a plane in a manner such as a 1D array and a 2D array. The 1D array is a manner in which a plurality of strip-shaped ultrasonic transducers 6 are apposed in a direction of the array. The 2D array is a manner in which a plurality of ultrasonic transducers 6 are apposed in a matrix shape. When voltage is applied to the ultrasonic transducer 6, it transmits ultrasonic waves in a direction in which the backing material 5 and the acoustic-matching layer 7 are layered. In addition, when receiving the waves reflected from the inside of the body of the subject, the ultrasonic transducer 6 converts the reflected waves into electrical signals by the piezoelectric effect and then outputs.

The electrode pair 9 applies voltage to the ultrasonic transducer 6 sandwiched thereby. Moreover, the electrical signals outputted by the ultrasonic transducer 6 having received the reflected waves flow through the electrode pair 9. The electrode pair 9 is provided for each of the plurality of ultrasonic transducers 6. A signal electrode of the electrode pair 9 is connected to a surface of each of the ultrasonic transducers 6, on the side of the backing material 5. A common (GND) electrode of the electrode pair is connected to a surface of the ultrasonic transducer 6, on the side of the acoustic-matching layer 7.

Of the ultrasonic waves transmitted by the ultrasonic transducer 6 and the reflected waves received thereby, the backing material 5 attenuates and absorbs ultrasonic-oscillation components that are not necessary for the ultrasonic diagnostic device D1 to extract an image. In addition, the backing material conveys heat produced in transmission of ultrasonic waves, to a heat-releasing member such as the cable 4. The coolant for cooling the ultrasonic wave transmitter/receiver 2, which is heated by oscillations of ultrasonic waves, circulates through the cable 4. The backing material 5 is composed of a material in which the attenuation rate of ultrasonic waves is high and a material with high thermal conductivity. For example, a material formed by mixing inorganic particle powders such as tungsten, ferrite and zinc oxide into synthetic rubber, epoxy resin, urethane or the like, is employed in general.

The acoustic-matching layer 7 suppresses reflection of ultrasonic waves on the surface of the body of the subject due to inconsistencies in acoustic impedance. This acoustic-matching layer 7 has immediate acoustic impedance between the acoustic impedance of the ultrasonic transducer 6 and the acoustic impedance of the surface of the body of the subject. This acoustic-matching layer 7 is composed of epoxy resin, plastics material, or the like. For the purpose of approximating in a stepwise fashion the acoustic impedance to that of the surface of the body of the subject, the acoustic-matching layer may be composed of a plurality of layers with different acoustic impedances.

The acoustic lens 8 comes in contact with the surface of the body of the subject, and intermediates the transmission/reception of ultrasonic waves. This acoustic lens 8 has a convex surface on the side of the subject. Ultrasonic waves oscillated by the ultrasonic transducer 6 are focused, whereby an acoustical focus is made at a predetermined depth.

In this ultrasonic probe 1, when voltage is applied from the electrode pairs 9 to the ultrasonic transducer 6, the ultrasonic transducer 6 oscillates ultrasonic waves by the piezoelectric effect. Heat is produced along with the oscillation of the ultrasonic waves. Unwanted ultrasonic waves transmitted toward the backing material 5 are attenuated by the backing material 5, and the produced heat is conveyed to the cable 4 by the backing material 5. The ultrasonic waves oscillated by the ultrasonic transducer 6 are radiated toward the subject by the acoustic-matching layer 7 without being reflected in the ultrasonic probe 1 or in the subject. The ultrasonic waves passed through the acoustic-matching layer 7 are focused by the acoustic lens 8 and radiated into the subject. The ultrasonic waves radiated into the subject bounce back toward the ultrasonic probe 1 as the reflected waves, due to the inconsistencies of the acoustic impedance in the subject. The inconsistencies of the acoustic impedance in the subject occur mainly on boundaries between sites in the subject. The ultrasonic transducer 6 captures the reflected waves. The ultrasonic transducer 6 converts the received reflected waves into electrical signals by the piezoelectric effect. The electrical signals outputted from the ultrasonic transducer 6 are outputted to the ultrasonic diagnostic device D1 via the electrode pair 9, the cable 4 and the connector 3.

In the ultrasonic diagnostic device D1, the electrical signals outputted from the ultrasonic probe 1 are A/D converted. To the A/D converted electrical signals, delay time necessary to determine the receiving directivity is given, and then, the signals are added. The electrical signals after the adding process are subjected to a band-pass filter process, and thereafter an envelope-detection process. A compression process by logarithmic conversion is applied to the detected data. Image-processing such as a volume rendering process or an MPR process is performed on the compression-processed detection data to generate image data such as three-dimensional image data or image data of any cross-section (MPR image data). This image data is outputted to a display monitor 18, and a three-dimensional image, an MPR image or the like is displayed on the screen of the display monitor 18.

A configuration for accomplishing a state-management part 27 of the ultrasonic probe 1 according to the present embodiment is shown in Fig. 3 through Fig. 5. Fig. 3 shows part of a circuit configuration of the ultrasonic probe 1 according to the present embodiment. The ultrasonic probe 1 comprises this circuit configuration. The connector 3 of the ultrasonic probe 1 incorporates a communication portion 10, an internal power supply 13, a computer portion 14 and a notifying portion 17. In addition, each of the ultrasonic wave transmitter/receiver 2 and the connector 3 incorporates a sensor 21. The communication portion 10 includes a connection circuit including a connector-pin 11 and a radio-communication circuit including an antenna 12. The computer portion 14 includes an arithmetic-control portion 15 and a storing portion 16. The notifying portion 17 includes a display monitor 18, a speaker 19 and an LED lamp 20. The communication portion 10 may be simply a connection circuit depending on a use pattern. In addition, the notifying portion 17 may be simply one of the monitor 18, the speaker 19 and the LED lamp 20.

The internal power supply 13 supplies electrical power to the communication portion 10, the computer portion 14, the sensor 21 and the notifying portion 17. In the ultrasonic diagnostic device D1, a power supply is normally disposed to the device main body. When the ultrasonic probe 1 is connected to the ultrasonic diagnostic device D1, the power supply of the device main body distributes power to the probe. The internal power supply 13 supplies electrical power when the ultrasonic probe 1 is detached. In other words, this ultrasonic probe 1 is capable of various operations even when detached from the ultrasonic diagnostic device D1.

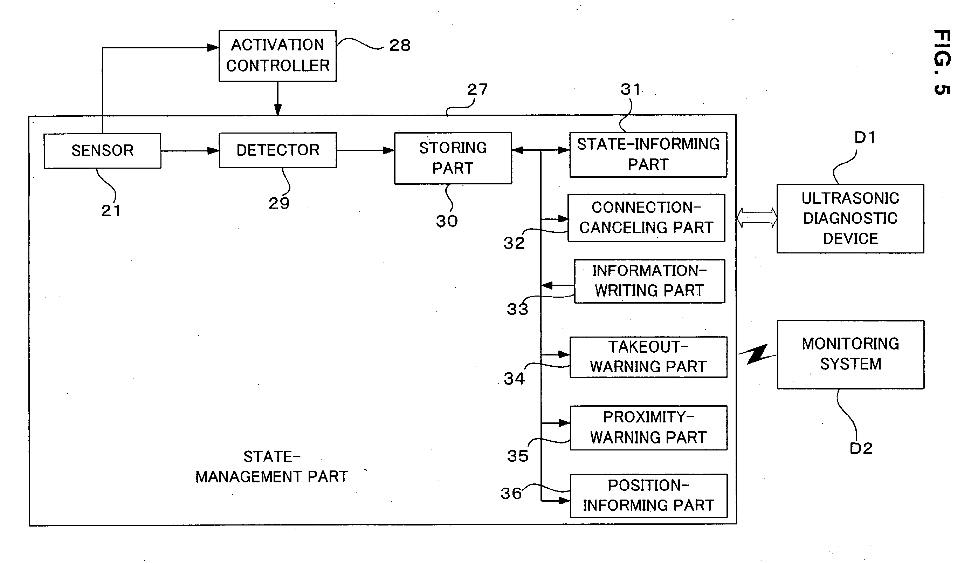

Although the internal power supply 13 may continuously feed power while the probe is detached from the ultrasonic diagnostic device D1, the probe comprises an activation controller 28 (*cf.* Fig. 5) in order to accomplish electrical-power saving. The activation controller 28 starts feeding power in response to sensing by a passive sensor that senses proximity of an object, or in response to reaching of predetermined timing by a timer. The activation controller may be configured so as to, when the ultrasonic probe 1 is in no danger of physical irritations such as shock, switch between starting or stopping power feeding in order to stop a radio-controlled monitoring operation by an external device. This internal power supply 13 has a function of checking the remaining amount of electromotive force. The ultrasonic probe 1 performs notification or communication for battery change, fuel addition or battery charge, when the remaining amount is below a rated value. As the internal power supply 13, a primary battery, a secondary battery or a fuel battery is employed. The primary battery is a chemical battery capable of only discharging of direct current, and is a battery in which a manganese battery, an alkali battery, an oxy alkali battery, a nickel manganese battery, a silver oxide battery, a mercury battery, a zinc-air battery, a lithium battery or the like is used as the positive electrode. The secondary battery is a chemical battery capable of discharging and charging, such as a lithium-ion battery and a nickel hydride battery. The fuel battery is a battery that is used by initiating a reaction contrary to electrolysis of water and taking outside the electric current generated at that time.

The communication portion 10 transmits/receives data to/from an external device, such as the ultrasonic diagnostic device D1 or the monitoring system D2 that is capable of radio communication. When connected to the ultrasonic diagnostic device D1, the communication portion transmits/receives data by wire via the connector-pin 11. When not physically connected to any external device, the communication portion transmits/receives data by radio via the antenna 12. The monitoring system D2 capable of radio communication includes, other than the ultrasonic diagnostic device D1, a mobile terminal such as a PDA and a mobile phone, and a computer connectable to a radio LAN. When connected to the ultrasonic diagnostic device D1 and used in ultrasonic diagnostics, the communication portion 10 receives electrical signals such as delayed data for supplying signal voltage to the electrode pair 9, from the ultrasonic diagnostic device D1 via the connector-pin 11. Moreover, the communication portion 10 transmits electrical signals of the reflected waves converted and outputted, to the ultrasonic diagnostic device D1 via the connector-pin 11. In addition, this communication portion 10 transmits various types of data generated by the computer portion 14 to the external device, and also receives various types of data from the external device and outputs to the computer portion 14.

Various types of data transmitted/received by the communication portion 10 are physical-irritation information, a cancel signal, a calling signal and handling information. The communication portion 10 transmits the physical-irritation information or the cancel signal to an external device. In addition, the communication portion 10 receives the calling signal or the handling information from the external device. The physical-irritation information represents the fact that physical irritation of a predetermined level or more has been applied to the ultrasonic probe 1 and the type of the physical irritation. Physical irritation occurring on the ultrasonic probe 1 includes shock that occurs outside or inside, changes in temperature, pressure, and changes in electrical resistance. The cancel signal is a signal for canceling communication for ultrasonic diagnostics with the ultrasonic diagnostic device D1 when physical irritation of a predetermined level or more has been applied to the ultrasonic probe 1. The calling signal is a signal for checking the position of the ultrasonic probe 1 from the external device. The handling information is data relating to the handling of the ultrasonic probe 1.

The arithmetic-control portion 15 causes the computer portion 14 to operate, by decoding and executing a program stored in the storing portion 16 as needed. The arithmetic-control portion 15 is a so-called MPU (Micro Processing Unit). The arithmetic-control portion 15 executes a program according to reaching of the predetermined timing as a result of timekeeping by a clock IC or according to a predetermined trigger, and controls the storing portion 16, the communication portion 10 and the notifying portion 17. The storing portion 16 is a so-called ROM (Read-Only Memory) or a RAM (Random-Access Memory) that stores programs and also reads and writes data. This computer portion 14 is driven by using as a trigger: reception of the electrical signals from the sensor 21; reception of a request for acquisition of stored data from the external device by the communication portion 10; reception of the calling signal by the communication portion 10; the reception of the handling information by the communication portion 10; connection to the ultrasonic diagnostic device D1 when physical irritation of a predetermined level or more is applied to the ultrasonic probe 1 in non-use; disconnection of communication with an external device by the communication portion 10; reception of a request for start or stop by the communication portion 1; or decrease in the remaining amount of the internal power supply 13.

The computer portion 14 is connected to each of the sensors 21 by a signal line. When receiving electrical signals from the sensor 21, the computer portion detects the electrical signals that represent physical irritation of a predetermined level or more. The physical irritation of the predetermined level or more refers to irritation outside the range of physical irritation occurring in a normal storage environment or normal conveying operation for the ultrasonic probe 1. For the computer portion 14, a threshold value is preset corresponding to the individual sensor 21. The threshold value is a boundary value between the level of physical irritation that may produce an abnormality in the ultrasonic probe 1 and the level of physical irritation that occurs in a normal storage environment or normal conveying operation. The electrical signals outputted from the sensor 21 are A/D converted to express the degree of strength of analog signals with digital values, and inputted into the computer portion 14. The computer portion 14 compares the values indicated by the electrical signals with the threshold value. When the value indicated by the electrical signals exceeds the threshold value, it is considered that the computer portion 14 has detected physical irritation of a predetermined level or more occurring in the ultrasonic probe 1.

When detecting physical irritation of a predetermined level or more, the computer portion 14 generates physical-irritation information and stores it in the storing portion 16. In addition, the computer portion 14 causes the communication portion 10 to transmit the physical-irritation information to an external device, and causes the notifying portion 17 to notify the physical-irritation information by sound, light or display.

Moreover, in response to a request for acquisition of the stored data from an external device, the computer portion 14 causes the communication portion 10 to transmit the physical-irritation information or handling information stored in the storing portion 16 to the external device.

When the communication portion 10 receives the calling signal, the computer portion 14 causes the notifying portion 17 to perform notification for checking the position of the ultrasonic probe 1.

When the communication portion 10 receives the handling information, the computer portion 14 causes the storing portion 16 to store the handling information. In a case in which the handling information is predetermined handling information such as waiting for sterilization, the computer portion causes the notifying portion 17 to give a warning, when an object such as a human body is detected to be in close proximity to the ultrasonic probe 1.

In a case in which physical irritation of a predetermined level or more has been applied to the ultrasonic probe 1 in use, the computer portion 14 generates a cancel signal and causes the communication portion 10 to transmit it, when the probe is connected to the ultrasonic diagnostic device D1.

The computer portion 14 monitors the communication portion 10 at every predetermined timing, and causes the notifying portion 17 to give a warning when the communication with an external device by the communication portion 10 becomes disconnected.

Upon receiving a start or stop request by the communication portion 10, the computer portion 14 stops electrical supply by controlling the internal power supply 13. When the internal power supply 13 senses decrease of the remaining amount of the internal power supply 13, the computer portion 14 transmits information for applying battery change, fuel addition or battery charge to the communication portion 10. In addition, the computer portion 14 may be configured so as to, when the internal power supply 13 senses decrease of the remaining amount of the internal power 13, cause the notifying portion 17 to display information for applying battery change, fuel addition or battery charge to the communication portion 10.

The sensor 21 monitors the state of the ultrasonic probe 1. The sensor 21 is a passive sensor: When the sensor 21 senses an event, the internal power supply 13 supplies electrical power to the computer portion 14, the communication portion 10 and the notifying portion 17, whereby the computer portion 14, the communication portion 10 and the notifying portion 17 are driven. Various types of sensors 21 are arranged in the ultrasonic probe 1 depending on the item being monitored.

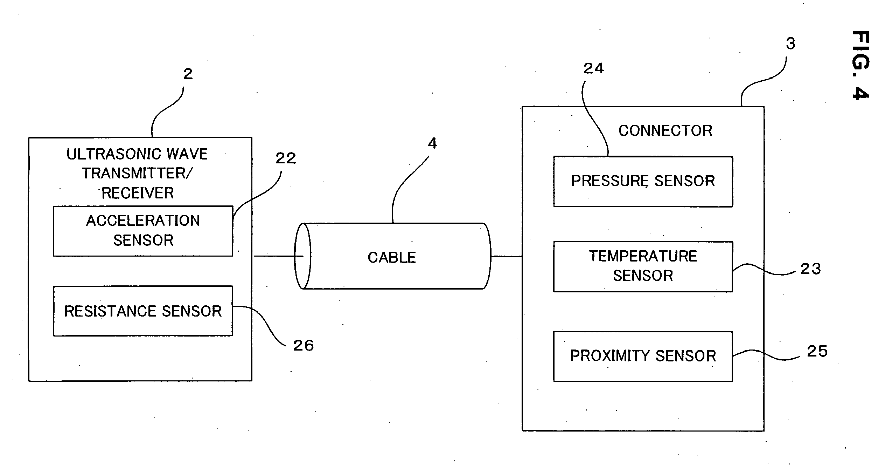

Fig. 4 shows a configuration of the sensor 21 placed in the ultrasonic probe 1. As shown in Fig. 4, an acceleration sensor 22 measuring acceleration, a temperature sensor 23 measuring temperature, a pressure sensor 24 measuring pressure, a proximity sensor 25 measuring the proximity of an object, and a resistance sensor 26 measuring resistance in a circuit, are placed in the ultrasonic probe 1.

The acceleration sensor 22 is placed in the vicinity of the ultrasonic transducer 6. Alternatively, the acceleration sensor may be disposed to a rigid body such as the backing material 5, which experiences shock of almost the same level as the ultrasonic transducer 6 experiences. For example, the acceleration sensor may be installed inside a space provided on the back face side of the backing material 5. This acceleration sensor 22 senses shock applied from outside to the ultrasonic probe 1. As the acceleration sensor 22, one capable of measuring the degree of strength of the shock is favorable. The sensor 21 that senses binary may be used as the acceleration sensor, but it becomes easier to set the threshold value when the sensor can measure the degree of strength of the shock. In addition, it is favorable to use, as the acceleration sensor 22, one that measures the vector direction of shock from three directions in total, which are the vertical direction to the plane on which the ultrasonic transducers 6 are apposed and the two-dimensional directions to the plane, in order to measure shock to the ultrasonic transducer 6 without omission.

In a configuration that the coolant for forced cooling is flown through the ultrasonic wave transmitter/receiver 2, the cable 4 and the connector 3 of the ultrasonic probe 1, the temperature sensor 23 senses low temperature out of concern of degradation due to freezing of the coolant.

The pressure sensor 24 senses the pressure of the coolant for forced cooling flowing in the cable 4. In addition, the pressure sensor 24 is disposed to a pump 3a that circulates the coolant in the connector 3. Thus, the pressure sensor senses deformation by measuring a change in pressure of the coolant when the cable is significantly deformed, and also senses leaks of the coolant by measuring a change in pressure caused by the leakage of the coolant.

The proximity sensor 25 is composed of a passive sensor, and outputs electrical signals to the internal power supply 13 and the computer portion 14 when an object such as a human body is in the proximity of the ultrasonic probe 1. The internal power supply 13 feeds power to the computer portion 14 in response to the electrical signals, and the computer portion 14 causes the notifying portion 17 to give a warning when predetermined handling information, such as waiting for sterilization, is stored.

The resistance sensor 26 is arranged in the circuit and senses a change in the insulation resistance. The insulation resistance is a resistance value between the power supply and the tip of the probe case coming in contact with the surface of the body of a subject. When this value becomes a predetermined value or less, electrical leakage might occur on the side of the patient.

The notifying portion 17 notifies various types of information by means of a screen display by the display monitor 18, sound by the speaker 19, or light by the LED lamp 20. The notifying portion 17 notifies based on state change or abnormality occurrence in the ultrasonic probe 1, proximity of a human body in a predetermined condition, unauthorized takeout of the ultrasonic probe 1, or a request for notification of the position of the ultrasonic probe 1. When physical irritation of a predetermined level or more is applied to the ultrasonic probe 1, the notifying part gives a warning by notifying the physical-irritation information stored in the storing portion 16 under the control of the computer portion 14. In a case where handling information, such as the ultrasonic probe 1 has been rejected in an electrical-leakage test or the ultrasonic probe 1 has been used in an examination for a diseased patient and is now waiting for sterilization and antisepsis, is written into the storing portion 16, the notifying part gives a warning under the control of the computer portion 14, when the proximity sensor 25 senses the proximity of an object such as a human body. When communication with the external device becomes disconnected in a state in which information on takeout is not stored in the storing portion 16, the notifying part gives a warning under the control of the computer portion 14. Upon receiving a calling signal from an external device, the notifying part announces the position of the ultrasonic probe 1 by lighting the LED lamp 20 under control by the computer portion 14.

Fig. 5 shows a functional configuration of the ultrasonic probe 1 comprising the communication portion 10, a detecting portion, the computer portion 14, the notifying portion 17, and the sensor 21. The ultrasonic probe 1 according to the present embodiment has the activation controller 28 and the state-management part 27. The state-management part 27 has the sensor 21 monitoring the state of the ultrasonic probe 1, a detector 29, a storing part 30, a state-informing part 31, a connection-canceling part 32, an information-writing part 33, a takeout-warning part 34, a proximity-warning part 35, and a position-informing part 36.

The activation controller 28 includes the internal power supply 13 and the computer portion 14, and activates the monitoring function of the ultrasonic probe 1 when the ultrasonic probe 1 is not connected to the ultrasonic diagnostic device D1. The clock IC such as a timer incorporated into the computer portion 14 times, and the activation controller makes the internal power supply 13 supply' electromotive force at a predetermined timing pre-stored in the storing portion 16. The activation controller causes the internal power supply 13 to supply electromotive force when the proximity sensor 25 senses the proximity of an object. In addition, when a start signal or a stop signal is transmitted from an external device via the communication portion 10, the activation controller makes the internal power supply 13 start or stop supply of electrical power. Incidentally, this activation controller 28 includes a dedicated activation circuit for determining the clocking of time including the clock IC and a predetermined timing, or a dedicated activation circuit for controlling the internal power supply 13 upon receiving electrical signals from the sensor 21, and a dedicated activation circuit for controlling the internal power supply 13 upon receiving a start signal or a stop signal in the computer portion 14, and a communication portion 10.

The detector 29 includes the computer portion 14, and detects a state change that may cause abnormalities in the ultrasonic probe 1, from electrical signals outputted from the sensor 29. In the detecting portion, a threshold value of acceleration, a threshold value of temperature, a threshold value of pressure, a threshold value of proximity, and a threshold value of resistance are preset corresponding to each sensor 21. When a value indicated by the electrical signals outputted from the sensor 21 exceeds the threshold stored in the storing portion 16, physical-irritation information is written into the storing part 30. The type of the threshold to be compared corresponds to the type of the sensor 21 having outputted the electrical signals, and it is distinguished with a signal line extending from the sensor 21. The detector 29 writes a pair of time information sensed by the sensor 21 and the type of the physical irritation added to the ultrasonic probe 1 as physical-irritation information into the storing part 30.

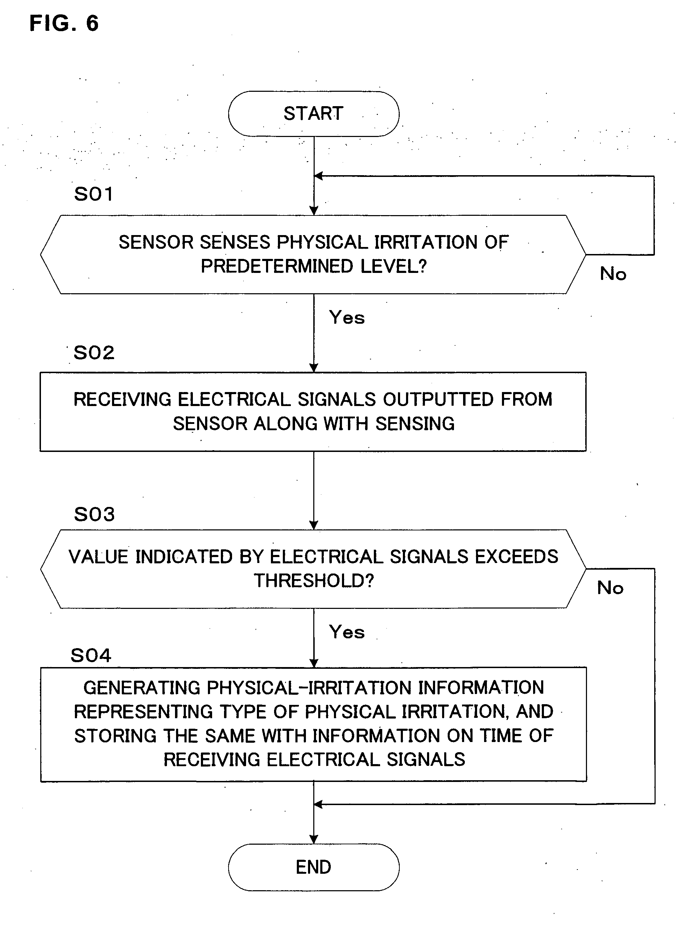

Fig. 6 is a flowchart showing an operation of the detector 29. First, when the sensor 21 senses physical irritation of a predetermined level such as a shock of a predetermined level or more and a temperature of a predetermined or more (S01, Yes), the detector 29 receives electrical signals outputted from the sensor 21 along with the sensing (S02). The detector 29 compares a value indicated by the electrical signals and the threshold (S03). When the value indicated by the electrical signals exceeds the threshold (S03, Yes), the detector 29 generates physical-irritation information representing the type of the physical irritation, and stores the electrical signals with information on reception time (S04). When the value indicated by the electrical signals is equal to or less than the threshold (S03, No), a process for sensing the physical event by the sensor 21 ends.

For example, the acceleration sensor 22 senses shock on the ultrasonic probe 1. At this moment, the acceleration sensor 22 outputs, to a signal line, electrical signals having a voltage value or current value that is proportional to the acceleration of the shock. These electrical signals are converted into digital signals that have a value, such as a voltage value or a current value, indicating the acceleration of the shock, and are outputted to the detector 29. Upon receiving the electrical signals from the acceleration sensor 22, the detector 29 compares them with the threshold for acceleration. When the value indicating the acceleration exceeds the threshold, the detector 29 reads the time information from the inside clock IC, generates physical-irritation information expressing that a shock of a predetermined level or more has been applied to the ultrasonic probe 1, and stores along with the time information.

The information-writing part 33 includes the computer portion 14, and writes the handling information transmitted from an external device, into the storing part 30. At the time of writing, the reception time of the handling information is attached to the handling information. A display button is arranged on the ultrasonic probe 1. When the display button is pressed down, the notifying portion 17 displays the contents of the handling information. The external device transmits information regarding handling of the ultrasonic probe 1, information permitting it to be taken out, and the like, as handling information. The information-writing part 33 writes this information as handling information into the storing part 30. The information regarding handling of the ultrasonic probe represents waiting for sterilization and antisepsis, waiting for repair, being rejected on an electrical leakage test, and the like, and the information for permitting to take out includes permission for taking out for sterilization and antisepsis, permission for taking out for repair, permission for taking out for an electrical leakage test, and the like.

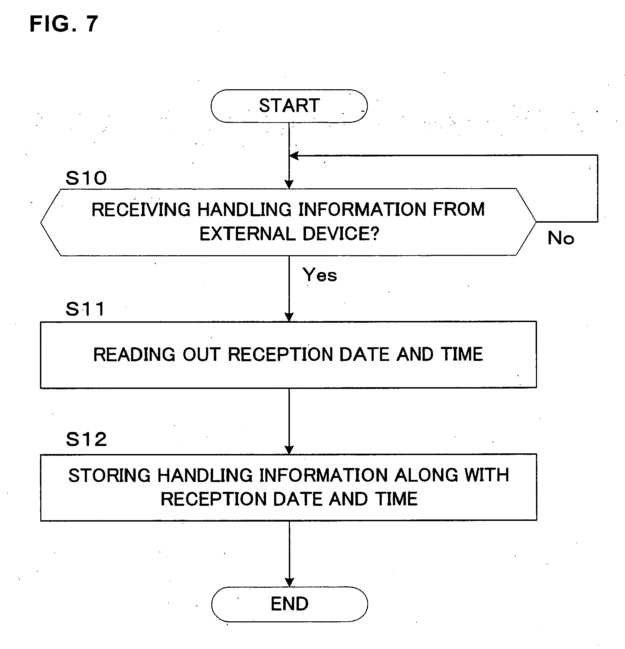

Fig. 7 is a flowchart showing an operation of writing handling information of this information-writing part 33. Upon receiving handling information from an external device (S10, Yes), the information-writing part 33 reads out the reception date and time from the clock IC (S11) and stores the handling information along with the reception date and time (S 12).

The storing part 30 includes a storing portion 16, and stores physical-irritation information and the handling information transmitted from the external device.

The state-informing part 31 includes the computer portion 14, and the communication portion 10 or notifying portion 17, and notifies physical-irritation information. The physical-irritation information is informed to' the external device via the communication portion 10, or the notifying portion 17 notifies the physical-irritation information. In addition, when handling information is stored on the storing part 30, this handling information is also informed. The physical-irritation information and handling information are transmitted when an acquisition request is transmitted by wire from the ultrasonic diagnostic device D1 and when an acquisition request is transmitted by radio from an external device, such as PDA, that is capable of radio communication. Besides that, it is also allowed to actively inform by using as a trigger the establishment of communication with an external device, or at a constant interval, or by using as a trigger the fact that physical-irritation information has been stored on the storing part 30. When physical-irritation information is notified by the notifying portion 17, the speaker 19 outputs by sound that physical irritation of a predetermined level or more has been applied, the LED lamp 20 emits a light color or blinking to indicate that physical irritation of a predetermined level or more has been applied, and the display monitor 18 displays the physical-irritation information thereon.

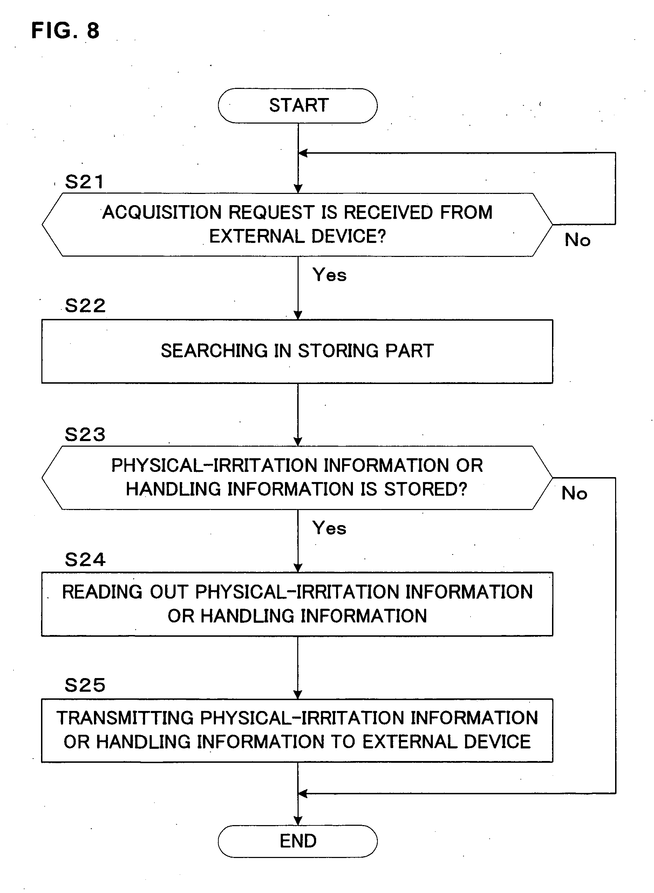

Fig. 8 is a flowchart showing an operation by the state-informing part 31 informing physical-irritation information and handling information. First, the state-informing part 31 receives an acquisition request from an external device such as the ultrasonic diagnostic device D1 or the monitoring system D2 (S21, Yes). Upon receiving the acquisition request, the state-informing part searches in the storing part 30 (S22). In a case where physical-irritation information or handling information is stored in the storing part 30 (S23, Yes), the state-informing part reads out the stored physical-irritation information or handling information (S24). Upon reading out the physical-irritation information or handling information, the state-informing part 31 transmits the physical-irritation information or handling information to the external device having made the acquisition request (S25). When physical-irritation information or handling information is not stored (S23, No), the state-informing part ends the informing process. In a case where the notifying portion 17 notifies the physical-irritation information or handling information, the notification is performed at S25.

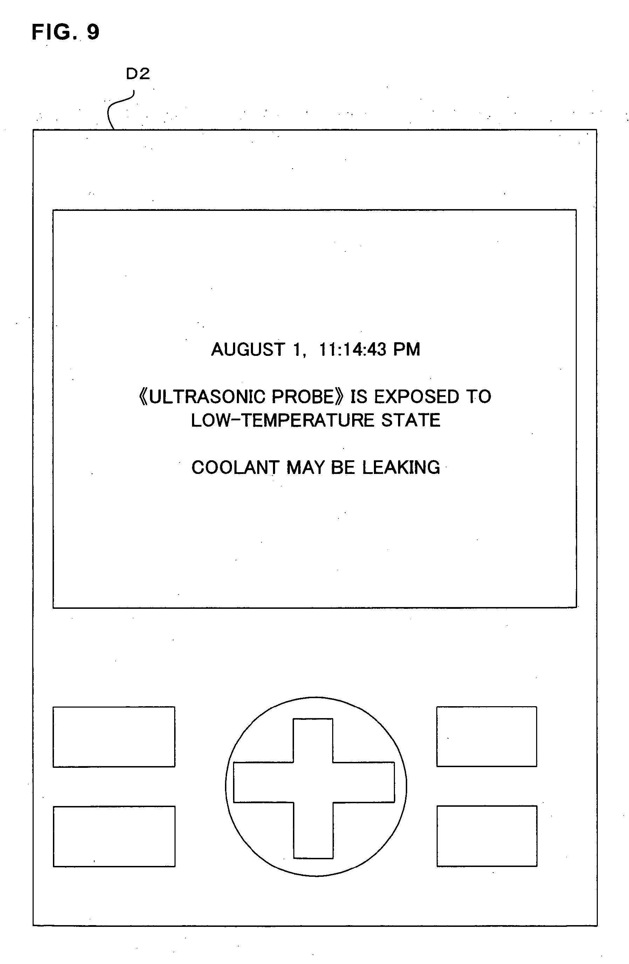

The external device having received the physical-irritation information or handling information displays the information on the display monitor 18. Consequently, an operator operating the external device can comprehend the state of the ultrasonic probe 1. Fig. 9 is a pattern diagram showing an example of a screen of the external apparatus, on which strings corresponding to the physical-irritation information or handling information transmitted by the state-informing part 31 are displayed. When the physical-irritation information is transmitted form the state-informing part 31, strings corresponding to the type of the physical-irritation information and the time information attached to the physical-irritation information are displayed on the screen of the external device. For example, when the physical-irritation information generated by sensing by the temperature sensor 23 is transmitted to the external device, a string "ultrasonic probe 1 is exposed to low-temperature state," indicating the sensing by the temperature sensor 23, a string "coolant may be leaking," indicating the cause thereof, and a string "August 1, 11: 14: 43 p.m.," indicating the time information, are displayed on the screen of the external device. In addition, when handling information is transmitted from the state-informing part 31, a string indicating the content of the handling information is displayed on the screen of the external device. For example, when handling information that indicates waiting for sterilization and antisepsis is transmitted to the external device, a string "this ultrasonic probe 1 is waiting for sterilization and antisepsis" is displayed on the screen of the external device.

The connection-canceling part 32 includes the computer portion 14 and the communication portion 10, and cancels data communication with the ultrasonic diagnostic device D1. In other words, the connection-canceling part transmits a cancel signal to the ultrasonic diagnostic device D1 so that a control signal for transmitting/receiving ultrasonic waves is not transmitted from the ultrasonic diagnostic device D1. The connection-canceling part 32 refers to the physical-irritation information stored in the storing part 30. When the physical-irritation information is stored in the storing part 30, a cancel signal is transmitted to the ultrasonic diagnostic device D1 via the communication portion 10.

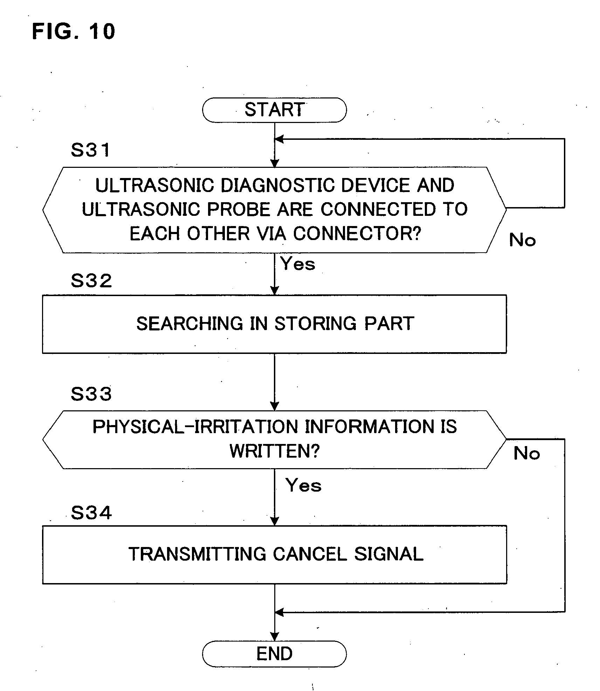

Fig. 10 is a flowchart showing an operation of canceling data communication by the connection-canceling part 32. First, the ultrasonic diagnostic device D1 and the ultrasonic probe 1 are connected to each other via the connector 3 (S31, Yes). The connection-canceling part 32 searches in the storing part 30 (S32). When physical-irritation information is written in the storing part 30 (S33, Yes), the connection-canceling part 32 generates a cancel signal and transmits the cancel signal to the ultrasonic diagnostic device D1 together with the physical-irritation information and the time information (S34). When physical-irritation information is not written in (S33, No), the cancel process is ended.

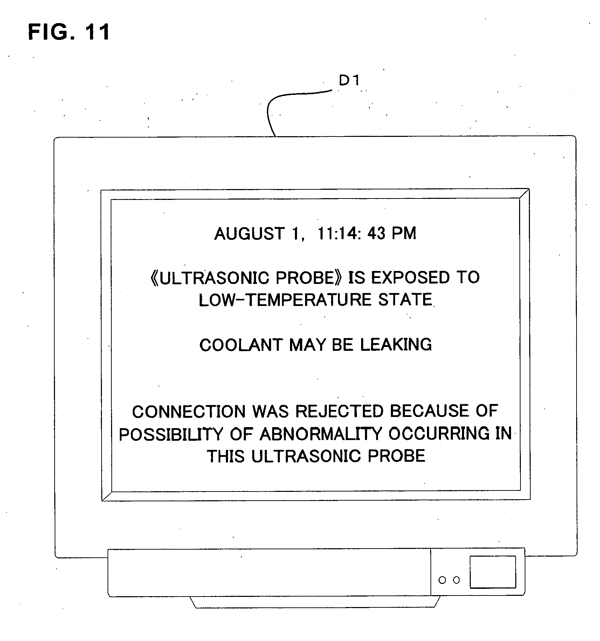

The ultrasonic diagnostic device D1 having received the cancel signal displays on the display monitor 18 the physical-irritation information and the fact that communication has been cut off, and cuts off the communication with the ultrasonic probe 1. This makes it possible to prevent ultrasonic diagnostics using the ultrasonic probe 1 with abnormalities, resulting in prevention of diagnostic errors or the like. Fig. 11 is a pattern diagram showing an example of a screen of the ultrasonic diagnostic device D1 having received a cancel signal. When a cancel signal is transmitted from the connection-canceling part 32, a string corresponding to the type of the physical-irritation information, time information attached to the physical-irritation information, and the fact that the communication has been cut off, are displayed on the screen of the ultrasonic diagnostic device D1. For example, when the physical-irritation information that has been generated based on sensing by the temperature sensor 23 is transmitted to the external device, a string "ultrasonic probe 1 is exposed to low-temperature state," indicating the sensing by the temperature sensor 23, a string "coolant may be leaking," indicating the cause thereof, a string "August 1, 11: 14: 43 p.m.," indicating the time information, and a string "connection was rejected because of possibility of abnormality occurring in this ultrasonic probe 1," indicating the fact that the communication has been cut off, are displayed on the screen of the ultrasonic diagnostic device D1.

The takeout-warning part 34 includes the computer portion 14 and the notifying portion 17, and gives a warning when the ultrasonic probe 1 is taken out without authority to take out. The warning against takeout is performed by the notifying portion 17. When the probe is taken out refers to when the data communication between the communication portion 10 and the external device becomes disconnected. The ultrasonic probe 1 is kept within an effective range for communication with an access point of a radio LAN. For example, in a safekeeping room for the ultrasonic probe 1, an access point is installed so that the effective range for data communication is within the safekeeping room. When taken out from the safekeeping room, the probe is out of the effective range for data communication with the access point and thereby data communication with the external device becomes disconnected, so that it can be recognized that the probe is taken out. The takeout-warning part 34 refers to information on permission of takeout stored in the storing part 30 when warning against takeout. When the information on permission of takeout is not stored in the storing part 30, the warning is given.

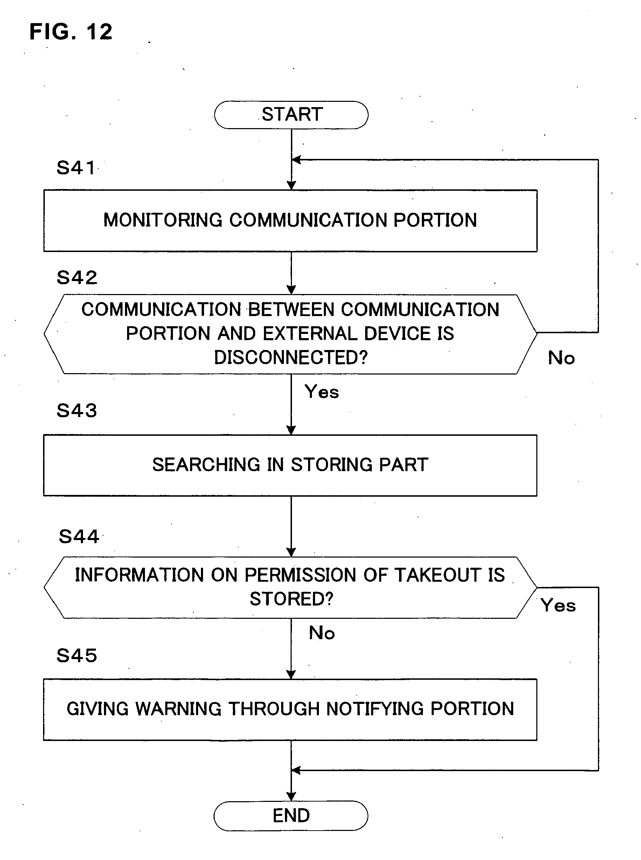

Fig. 12 is a flowchart showing an operation of giving a warning against takeout by the takeout-warning part 34. The takeout-warning part 34 monitors the communication portion 10 at every predetermined time (S41). When the communication between the communication portion 10 and the external device is disconnected (S42) (i.e. when the connection is cut off), the takeout-warning part 34 searches in the storing part 30 (S43). When information on permission of takeout is stored in the storing part 30 (S44, Yes), the takeout-warning part 34 ends the process of warning against takeout. On the other hand, when information on permission of takeout is not stored in the storing part 30 (S44, No), the takeout-warning part 34 gives a warning through the notifying portion 17 (S45).

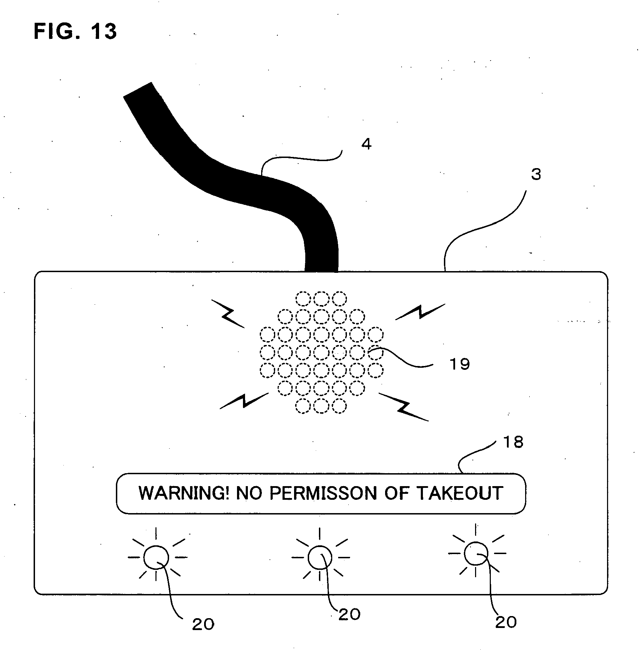

Fig. 13 is a pattern diagram showing a state in which the ultrasonic probe 1 is warning against unauthorized takeout. The speaker 19 sounds a warning, the LED lamp 20 emits a light or blinking color indicating unauthorized takeout, and the display monitor 18 displays a warning. The proximity-warning part 35 includes the computer portion 14 and the notifying portion 17, and gives a warning when the proximity sensor 25 senses an object such as a human body while the ultrasonic probe 1 is in a predetermined state. When electrical signals are outputted from the proximity sensor 25, the proximity-warning part 35 refers to handling information stored in the storing part 30. When information indicating adverse effects on a human body, such as waiting for sterilization and antisepsis, is stored as handling information, the proximity-warning part 35 gives a warning through the notifying portion 17.

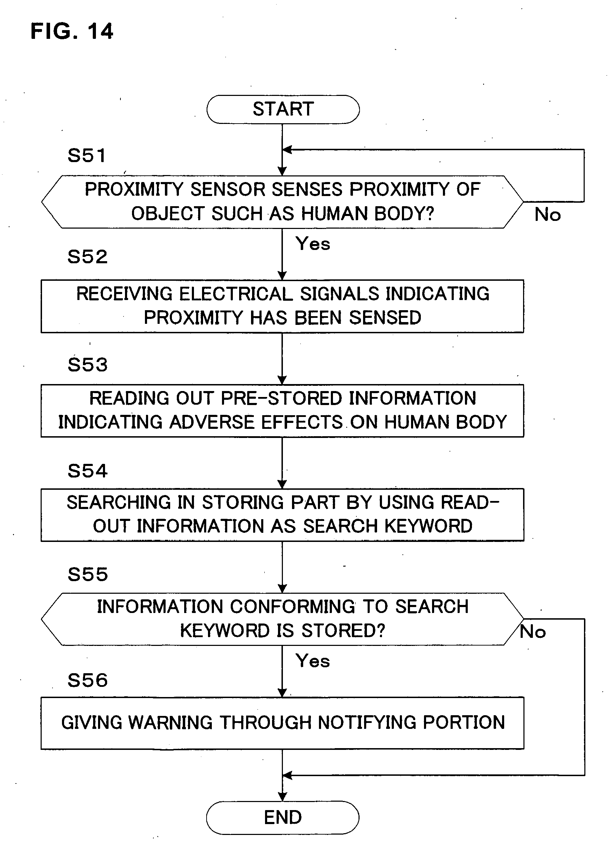

Fig. 14 is a flowchart showing an operation of warning against proximity by the proximity-warning part 35. First, the proximity sensor 25 senses proximity of an object such as a human body (S51, Yes). When the proximity sensor 25 outputs electrical signals indicating that proximity has been sensed and the proximity-warning part 35 receives the electrical signals indicating that proximity has been sensed (S52), the proximity-warning part 35 reads out the pre-stored information indicating adverse effects on a human body (S53). The proximity-warning part 35 searches in the storing part 30 by using the read-out information as a search keyword (S54). When information conforming to the search keyword is stored in the storing part 30 (i.e. when handling information that indicates adverse effects on a human body is stored in the storing part 30 (S55, Yes)), the proximity-warning part 35 gives a warning through the notifying portion 17 (S56). On the other hand, when handling information that indicates adverse effects on a human body is not stored (S55, No), the proximity warning process is ended.

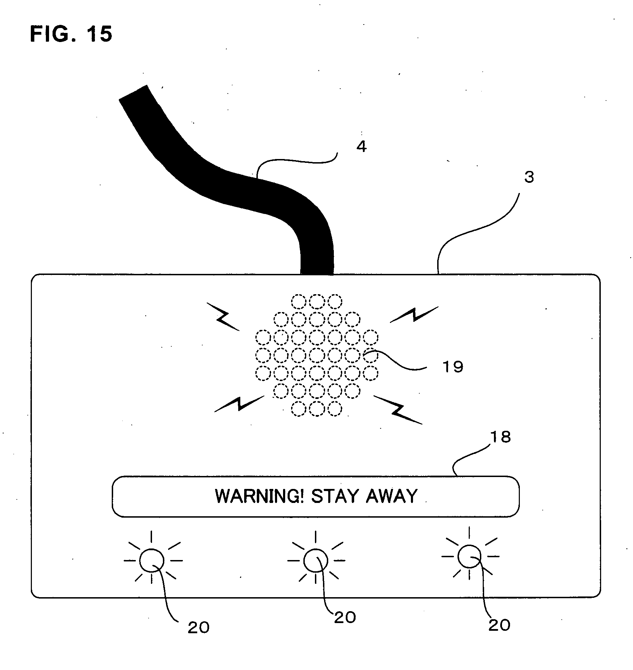

Fig. 15 is a pattern diagram showing a state in which the ultrasonic probe 1 is warning against proximity. The speaker 19 sounds a warning, the LED lamp 20 emits a light or blinking color that indicates danger, and the display monitor 18 displays a warning. The position-informing part 36 includes the computer portion 14, the communication portion 10 and the notifying portion 17. The position-informing part 36 notifies the position of the ultrasonic probe 1 through the notifying portion 17 when receiving a calling signal from an external device via the communication portion 10. The calling signal transmitted from the external device includes identification information inherent in individual ultrasonic probe 1. The identification information of the ultrasonic probe 1 is pre-stored in the storing portion 16. The position-informing part 36 notifies the position through the notifying portion 17 when receiving a calling signal that includes its own identification information.

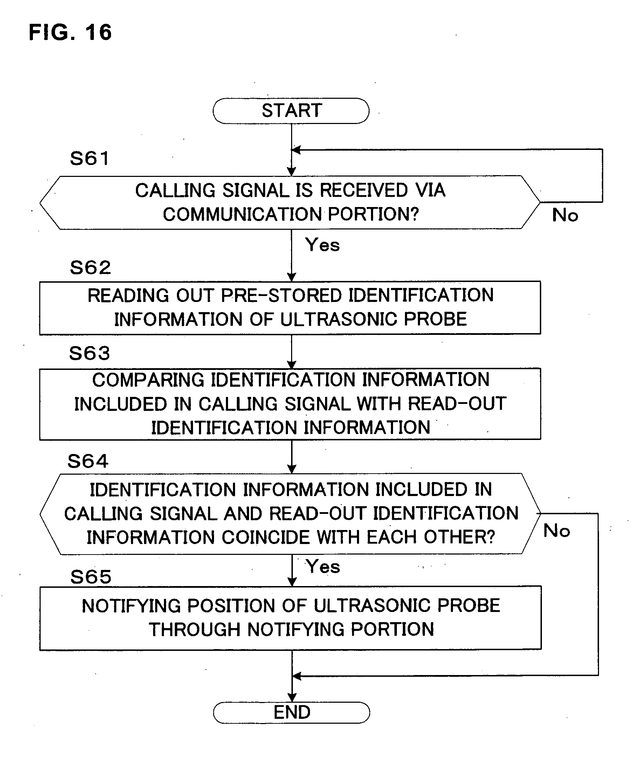

Fig. 16 is a flowchart showing an operation of informing the position by the position-informing part 36. First, when receiving a calling signal via the communication portion 10 (S61, Yes), the position-informing part 36 reads out the pre-stored identification information of the ultrasonic probe 1 (S62). The position-informing part 36 then compares the identification information included in the calling signal with the read-out identification information (S63). When the identification information included in the calling signal and the read-out identification information coincide with each other (S64, Yes), the position-informing part 36 notifies the position of the ultrasonic probe 1 through the notifying portion 17 (S65). On the other hand, when the identification information included in the calling signal and the read-out identification information do not coincide with each other, the process of notifying the position is ended.

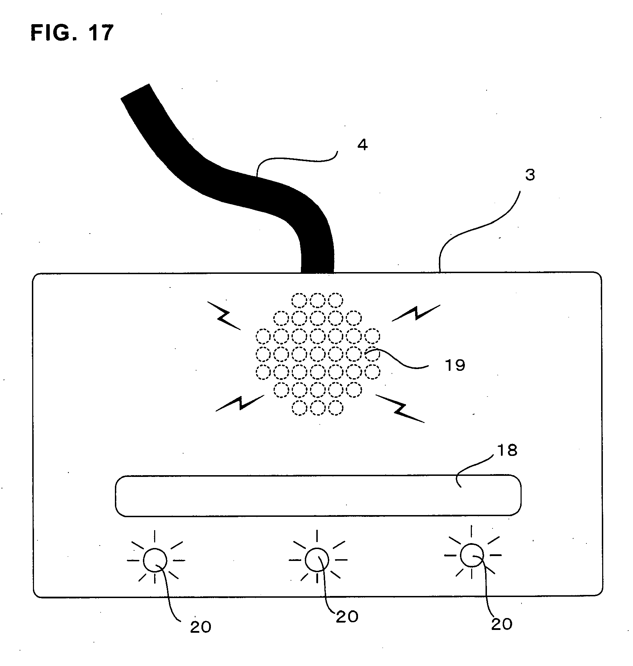

Fig. 17 is a pattern diagram showing a state in which the ultrasonic probe 1 is notifying the position. The speaker 19 emits a sound for announcing the position, the LED lamp 20 emits a light or blinking color indicating the position, the display monitor 18 lights a backlight thereof.

In the present embodiment, only when the detector 29 determines that there has been a state change that might cause an abnormality in the ultrasonic probe 1, physical-irritation information indicating the state change is stored in the storing part 30 and transmitted to an external device. Alternatively, regardless of whatever the result of sensing by the sensor 21 is, all the results of sensing by the sensor 21 may be stored in the storing part 30 and transmitted to the external device.

Further, the sensitivity of the sensor 21 may be adjusted so that, only when there has been a state change that might cause an abnormality in the ultrasonic probe 1, the sensor 21 senses the state. In this case, it is possible to omit the detecting portion, and it is also allowable to store the result of sensing by the sensor 21 in the storing part 30 and consider the sensing result as physical-irritation information.

Furthermore, the monitoring system D2 may have a function like the takeout-warning part 34. Information on permission of takeout is written into a storage area of the monitoring system D2, as in storing part 30. If the information on permission of takeout is not written in when communication with the ultrasonic probe 1 becomes disconnected or when the communication cannot be established, the monitoring system D2 gives a warning that the probe has been taken out without permission, through a speaker or display monitor of the monitoring system D2.

As described above, the ultrasonic probe 1 according to the present embodiment comprises the internal power supply 13 capable of power supply in a state in which the probe is detached from the ultrasonic diagnostic device D1, and the state-management part 27 fed by the internal power supply 13 and managing the state of the main body of the ultrasonic probe 1. Consequently, it is possible to manage the state of the ultrasonic probe 1 in non-use. The state-management part 27 senses physical irritations occurring in the main body of the ultrasonic probe 1 by using the sensor 21 and detects physical irritation of a predetermined level or more based on the result of sensing by the sensor 21, thereby enabling the specification of the factors involved in abnormality occurrence. In addition, the state-management part 27 transmits by radio to an external device that physical irritation of a predetermined level or more has been applied, whereby it is possible to comprehend the risk of abnormality occurrence without omission.

Further, the state-management part 27 notifies by sound, light or display the fact that physical irritation of a predetermined level or more has been applied. This enables a handler to comprehend what type of handling may cause abnormalities and to learn an appropriate way of handling.

Furthermore, when physical irritation of a predetermined level or more is detected, the state-management part 27 outputs a cancel signal of the signal transmission/reception to the ultrasonic diagnostic device D1 when the connector 3 is connected to the ultrasonic diagnostic device D1. This makes it possible to prevent the probe from being used in diagnosis without the operator being aware of abnormality occurrences and to prevent false diagnoses using inaccurate diagnostic information or re-diagnoses.

Further, the state-management part 27 receives handling information of the main body of the ultrasonic probe 1 from an external device. This handling information is displayed on the display monitor 18. This makes it possible to immediately comprehend handling information, such as whether the ultrasonic probe 1 is currently waiting for sterilization or maintenance. In addition, when the proximity sensor 25 measures the proximity of an object such as a human body, and receiving handling information that indicates an adverse affect on human bodies, such as waiting for sterilization or electrical leakage, a warning is given with sound, light or display. This makes it possible to prevent a disaster caused by using an ultrasonic probe 1 that may adversely affect a human body.

Further, the state-management part 27 gives a warning with sound, light or display when disconnection of communication with the external device is detected. This makes it possible to issue a warning, when the ultrasonic probe 1 is taken out without permission, to the person taking it out, thereby offering an antitheft effect.

Further, the state-management part 27 notifies the position of the main body of the ultrasonic probe 1 by using sound, light or display when receiving a calling signal from an external device. This makes it possible to easily select a desired ultrasonic probe 1 from among a plurality of ultrasonic probes 1, and to use in diagnosis an ultrasonic probe 1 that has no abnormalities.

Further, the probe further comprises the activation controller 28 for controlling the timing of power supply from the internal power supply 13 to the state-management part 27. This makes it possible to hold down the power consumption of the internal power supply 13, and achieve high convenience.

It is explicitly stated that all features disclosed in the description and/or the claims are intended to be disclosed separately and independently from each other for the purpose of original disclosure as well as for the purpose of restricting the claimed invention independent of the composition of the features in the embodiments and/or the claims. It is explicitly stated that all value ranges or indications of groups of entities disclose every possible intermediate value or intermediate entity for the purpose of original disclosure as well as for the purpose of restricting the claimed invention, in particular as limits of value ranges.

## Claims

1. An ultrasonic probe comprising:
an ultrasonic wave transmitter/receiver configured to transmit/receive ultrasonic waves;
a connector connected to an ultrasonic diagnostic device and configured to mediate signal transmission/reception to/from the ultrasonic wave transmitter/receiver;
a state-management part configured to manage a state of the ultrasonic probe; and
an internal power supply configured to be capable of supplying electrical power to the state-management part in a state in which the connector is detached from the ultrasonic diagnostic device.

2. The ultrasonic probe according to Claim 1, wherein the state-management part includes:
a sensor configured to sense physical irritations received by the ultrasonic probe; and
a detector configured to detect, based on a result of sensing by the sensor, the physical irritations of a predetermined level or more.

3. The ultrasonic probe according to Claim 2, wherein the sensor is at least one of a sensor configured to measure acceleration, a sensor configured to measure temperature, a sensor configured to measure pressure, and a sensor configured to measure insulation resistance of the ultrasonic probe.

4. The ultrasonic probe according to Claim 2, wherein the state-management part further includes a radio-communication part configured to transmit by radio a detection result detected by the detector to an external device.

5. The ultrasonic probe according to Claim 2, wherein the state-management part further includes a notifying part configured to notify the detection result detected by the detector, by sound, light or display.

6. The ultrasonic probe according to Claim 2, wherein the state-management part further includes a communication part configured to, when the detector detects the physical irritations of the predetermined level or more, output a cancel signal of the signal transmission/reception to the ultrasonic diagnostic device when the connector is connected to the ultrasonic diagnostic device.

7. The ultrasonic probe according to Claim 1, wherein the state-management part includes a communication part configured to receive handling information of the ultrasonic probe from an external device.

8. The ultrasonic probe according to Claim 7, wherein the state-management part further includes a notifying part configured to display the handling information received by the communication part.

9. The ultrasonic probe according to Claim 7, wherein the state-management part further includes:
a sensor configured to measure proximity of an object; and
a notifying part configured to, when the communication part receives a predetermined piece of the handling information and the sensor measures the proximity of the object, give a warning by sound, light or display.

10. The ultrasonic probe according to Claim 1, wherein the state-management part further includes:
a radio-communication part configured to communicate by radio with an external device; and
a notifying part configured to, when radio communication between the radio-communication part and the external device becomes disconnected, give a warning by sound, light or display.

11. The ultrasonic probe according to Claim 1, wherein the state-management part further includes:
a radio-communication part configured to receive a calling signal from an external device; and
a notifying part configured to, when the radio-communication part receives the calling signal, notify a position of the ultrasonic probe by sound, light or display.

12. The ultrasonic probe according to Claim 1, further comprising an activation controller configured to control timing of power feeding from the internal power supply to the state-management part.

13. The ultrasonic probe according to Claim 12, wherein the activation controller includes a timer configured to clock timing of supply of electromotive force from the internal power supply to the ultrasonic probe.

14. The ultrasonic probe according to Claim 12, wherein:
the activation controller includes a passive sensor; and
power is fed from the internal power supply to the state-management part by taking a cue from the sensing by the passive sensor.

15. The ultrasonic probe according to Claim 12, wherein:
the activation controller includes a radio-communication part configured to receive a feed-start signal or feed-stop signal from an external device; and
power feeding from the internal power supply to the state-management part is switched in accordance with the feed-start signal or feed-stop signal that the radio-communication part receives from the external device.

16. An ultrasonic diagnostic device comprising:
an ultrasonic probe removably connected to a device main body and configured to transmit/receive ultrasonic waves to/from an inside of a subject while transmitting/receiving signals for ultrasonic diagnostics to/from the device main body;
an image processor configured to generate an image of the inside of the subject based on the received ultrasonic waves; and
a device power supply disposed to the device main body and configured to supply electrical power to the ultrasonic probe when the ultrasonic probe and the device main body are connected to each other, wherein the ultrasonic probe comprises:
a state-management part configured to manage a state of the ultrasonic probe; and
an internal power supply configured to be capable of supplying electrical power to the state-management part in a state in which the ultrasonic probe is detached from the device main body.

17. The ultrasonic diagnostic device according to Claim 16, wherein the state-management part includes:
a sensor configured to sense physical irritations received by the ultrasonic probe;
a detector configured to detect, based on a result of sensing by the sensor, the physical irritations of a predetermined level or more; and
a communication part configured to, when the detector detects the physical irritations of the predetermined level or more, output a cancel signal of the signal transmission/reception for the ultrasonic diagnostics to the device main body when the ultrasonic probe is connected to the device main body.

18. An ultrasonic-probe-monitoring system having an ultrasonic probe and an external device, wherein:
the ultrasonic probe comprises:
a sensor configured to sense physical irritations received by the ultrasonic probe;
a detector configured to detect, based on a result of sensing by the sensor, the physical irritations of a predetermined level or more;
a radio-communication part configured to transmit by radio the detection result detected by the detector to the external device; and
an internal power supply configured to be capable of supplying electrical power to the inside of the ultrasonic probe in a state in which the probe is detached from an ultrasonic diagnostic device; and the external device comprises:
a radio-communication part configured to receive the detection result; and
a notifying part configured to notify the received detection result.

19. A method of state management of an ultrasonic probe having a state-management part configured to manage a state of the ultrasonic probe and an internal power supply, wherein:
electrical power is supplied from the internal power supply to the state-management part when the ultrasonic probe is detached from an ultrasonic diagnostic device; and
the state-management part manages the state of the ultrasonic probe in a state in which the ultrasonic probe is detached from the ultrasonic diagnostic device, upon receiving supply of the electrical power.
